# EUROPÄISCHE PATENTSCHRIFT

(11) **EP 1 559 402 B1**
(45) Veröffentlichungstag und Bekanntmachung des Hinweises auf die Patenterteilung: **15.08.2007**
(21) Anmeldenummer: 05000097.5
(22) Anmeldetag: 05.01.2005
(51) Int. Cl.: A61K 8/92, A61K 8/891, A61Q 5/06

(54) **Haarwachsprodukt aus Silikonwachs, silikonfreiem Wachs und Ölen**
Hairwax product made of silicone wax, a silicone-free wax and oils.
Cire pour les cheveux comprenant une cire de silicone, une cire exempte de silicone et des huiles.

(30) Priorität: 28.01.2004 DE 102004004155
(43) Veröffentlichungstag der Anmeldung: 03.08.2005
(73) Patentinhaber: Wella Aktiengesellschaft, 64274 Darmstadt (DE)
(72) Erfinder: Loifenfeld, Marina, 60316 Frankfurt am Main (DE); Stein, Bernd, 63768 Hösbach (DE); Franzke, Michael, Dr., 64380 Rossdorf (DE)

(56) Entgegenhaltungen:
- EP-A- 0 760 235
- EP-A- 0 868 898
- EP-A- 1 197 201
- EP-A- 1 266 650
- WO-A-96/15761
- WO-A-20/04009037
- DE-B- 1 617 421
- DE-U- 20 016 826
- US-A- 2002 106 385
- US-A1- 2003 211 070
- US-B- 6 027 738

## Beschreibung

Gegenstand der Erfindung ist ein Wachsprodukt zur Behandlung oder Erstellung der menschlichen Frisur. Das Wachsprodukt enthält eine Zusammensetzung mit einem Gehalt an einem bei Raumtemperatur festen, silikonfreien Wachs, einem bei Raumtemperatur flüssigen Öl und einem Silikonwachs mit einem Schmelzpunkt in der Nähe der menschlichen Körpertemperatur.

Stylingwachs-Zusammensetzungen sind bekannte Produkte zur Haarbehandlung. Sie finden insbesondere Anwendung, um kurzes bis mittellanges Haar trendgerecht in Form zu bringen und der Frisur Halt, Stand und Festigung sowie Glanz zu verleihen. Auch lassen sich mit Haarwachsen Konturen und Texture in der Frisur erzeugen. Herkömmliche Haarwachse werden üblicherweise in Tiegeln angeboten und beruhen auf folgendem Wirkungsprinzip: Die Entnahme der Produktmasse erfolgt mit den Fingern. Das Wachs wird in den Handflächen verteilt und durch die Handwärme aufgeschmolzen oder zumindest stark erweicht. Durch die Erweichung oder Aufschmelzung wird die Einarbeitung des ansonsten zu festen Wachses in das Haar ermöglicht. Im erweichten oder mehr oder weniger flüssigen Zustand wird das Wachs in das Haar eingearbeitet. Im Haar kühlt es ab und erreicht wieder seine Ausgangskonsistenz. Dabei erhärtet es und die gestaltete Frisur erhält Stabilität und Halt sowie häufig einen leichten Wet-Look. Herkömmliche Haarwachsprodukte, wie sie derzeit angeboten werden, sind in der Regel auf der Basis von hydrophoben, nicht-silikonhaltigen Wachsen, Fetten und Ölen aufgebaut. Sie enthalten einen großen Anteil an hydrophoben Stoffen wie z.B. pflanzlichen oder tierischen Wachsen, Fettsäureestern, Fettalkoholen etc.. Hauptbestandteile und Hauptwirkstoffe sind hydrophobe Wachse wie z.B. Ozokerit, Candelillawachs, Bienenwachs, Carnaubawachs etc. Derartige Produkte haben den Nachteil, dass sie bei Anwendung auf dem Haar außer den gewünschten Effekten eine relativ hohe, unerwünschte Klebrigkeit hinterlassen.

EP 868 898 A1 offenbart eine Dispersionszusammensetzung, die ein amphoteres und/oder semipolares Netzmittel, ein nichtionisches Netzmittel sowie ein Wachs enthält.

Die Aufgabe bestand darin, ein festes Haarwachsprodukt zur Verfügung zu stellen, welches beim Verreiben mit den Händen zu einer erweichten oder flüssigen, glatten, nicht oder möglichst gering klebrigen Konsistenz schmilzt und wobei das Haar nach dem Aufbringen des Produktes natürlich und beweglich bleibt und Locken oder einzelne Haarsträhnen betont werden können.

Darüberhinaus soll das Produkt gut dem Verpackungsmittel entnehmbar und gut verarbeitbar sein und gute Anwendungseigenschaften auf dem Haar aufweisen, insbesondere gute Einarbeitbarkeit, gute Verteilbarkeit und gute Frisurendefinition und der Frisur Glanz, Halt, Festigung bzw. Stand verleihen.

Es wurde nun gefunden, dass die Anforderungen erfüllt werden durch ein festes Haarwachsprodukt zur Behandlung oder Erstellung der menschlichen Frisur, welches ein bei Raumtemperatur festes, silikonfreies Wachs, eine bei Raumtemperatur flüssige Ölverbindung und ein Silikonwachs mit einem Schmelzpunkt in der Nähe der menschlichen Körpertemperatur enthält. Gegenstand der Erfindung ist daher die Verwendung eines Haarwachsproduktes mit fester, wachsartiger Konsistenz zur Behandlung oder Erstellung der menschlichen Frisur mit einem Gehalt an
(A) mindestens einem silikonfreien Wachs,
(B) mindestens einem bei 25°C flüssigen, hydrophoben Öl und
(C) mindestens einem Silikonwachs mit Schmelzpunkt im Bereich von 20 bis 45 °C.

Die wachsartige Konsistenz ist dadurch gekennzeichnet, dass die Nadelpenetrationszahl (Maßeinheit 0,1 mm, Prüfgewicht inkl. Führungsstange 25 g, Prüfdauer 5 s, Prüftemperatur 20°C; Geometrie des Hohlkonus wie in DIN 51 580) vorzugsweise größer oder gleich 10, insbesondere von 10 bis 60, von 15 bis 55 oder von 25 bis 50 beträgt und dass das Produkt einen Erstarrungspunkt aufweist, welcher vorzugsweise größer oder gleich 30°C ist und besonders bevorzugt im Bereich von 40 bis 55°C liegt.

### Silikonfreie Wachse

Die silikonfreien Wachse werden vorzugsweise in einer Menge von 10 bis 40 Gew,%, insbesondere von 15 bis 30 oder von 15 bis 25 Gew.% eingesetzt. Sie haben einen Erstarrungspunkt von vorzugsweise oberhalb 40°C oder oberhalb 55°C. Die Nadelpenetrationszahl liegt vorzugsweise im Bereich von 2 bis 70, insbesondere von 3 bis 40. Vorzugsweise ist mindestens ein Wachs enthalten, welches eine Nadelpenetrationszahl kleiner 40, besonders bevorzugt kleiner 20 aufweist.

Geeignete silikonfreie Wachse sind z.B. tierische Wachse, pflanzliche Wachse, mineralische Wachse, synthetische Wachse, mikrokristalline Wachse, makrokristalline Wachse, Paraffinwachse, Ozokerit, Montanwachsen, Fischer-Tropsch-Wachse, Polyolefinwachse (z.B. Polyethylen, Polybuten und ähnliche), Bienenwachs, Wollwachs (Lanolin) und dessen Derivate wie z.B. Wollwachsalkohole, Candelillawachs, Carnaubawachs, Japanwachs, gehärtete Fette, Fettsäureester und Fettsäureglyceride. Eine bevorzugte Ausführungsform enthält als silikonfreie Wachse Kohlenwasserstoffwachse, z.B. Ozokerit oder Ceresin, insbesondere mit einer Nadelpenetrationszahl kleiner 20.

### Flüssige, hydrophobe Öle

Die flüssigen, hydrophoben Öle werden vorzugsweise in einer Menge von 20 bis 80 Gew,%, insbesondere von 30 bis 70 oder von 40 bis 55 Gew.% eingesetzt. Sie haben einen Schmelzpunkt von kleiner oder gleich 25°C und einen Siedepunkt von vorzugsweise über 250 °C, insbesondere über 300 °C. Hierfür kann prinzipiell jedes dem Fachmann allgemein bekannte Öl eingesetzt werden. Vorzugsweise handelt es sich um Silikonverbindungen. Geeignete Silikonöle sind z.B. lineare Polydimethylsiloxane (Dimethicone), cyclische Polydimethylsiloxanen (Cyclomethicone), hydroxysubstituierte Polydimethylsiloxane (Dimethiconole), aminosubstituierte Silikone (z.B. Amodimethicone) und mit aromatischen Gruppen substituierte Siloxane (z.B. phenylierte Silikone, Polyphenylmethylsiloxane, Phenyltrimethicone).

Zusätzlich oder alternativ können auch hydrophobe, silikonfreie Öle eingesetzt werden. In Frage kommen pflanzliche oder tierische Öle, Mineralöle (Paraffinum liquidum) oder deren Mischungen. Geeignet sind Kohlenwasserstofföle, z.B. Paraffin- oder Isoparaffinöle, Squalan, Öle aus Fettsäuren und Polyolen, insbesondere Triglyceride. Geeignete pflanzliche Öle sind z.B. Sonnenblumenöl, Kokosöl, Rizinusöl, Lanolinöl, Jojobaöl, Maisöl, Sojaöl.

### Silikonwachse

Die Silikonwachse werden vorzugsweise in einer Menge von 5 bis 40 Gew,%, insbesondere von 10 bis 25 oder von 10 bis 20 Gew.% eingesetzt. Sie haben einen Schmelzpunkt im Bereich von 20 bis 45 °C, vorzugsweise im Bereich von 25 bis 40 oder bis 35°C.
Die Silikonwachse sind vorzugsweise ausgewählt aus Alkylmethyl-dimethylsiloxanen, insbesondere solchen, bei denen die Alkylgruppe mindestens 8 C-Atome, z.B. 16 bis 20 C-Atome aufweist. Alkylmethyl-dimethylsiloxane haben die allgemeine Formel

Me₃SiO- [Si(Me)R-O-]ₓ-[SiMe₂-O-]_{y}-SiMe₃

wobei Me eine Methylgruppe und R eine Alkylgruppe mit vorzugsweise 8 bis 22 C-Atomen bedeuten und x und y Zahlen größer 0 sind.
Eine bevorzugte Ausführungsform enthält Stearylmethyldimethylsiloxan (INCI: Stearyl Dimethicone). Handelsprodukte sind z.B. Abil Wax 9800, DC 2503 Wax, SilCare 41M65, Silsoft W-18 und Belsil SDM 5055.

### Emulgatoren

In einer bevorzugten Ausführungsform enthält das erfindungsgemäße Haarwachsprodukt zusätzlich mindestens einen Emulgator. Die Emulgatoren sind vorzugsweise in einer Menge von 0,1 bis 30 Gew.%, von 0,5 bis 25 Gew.% oder von 3 bis 20 Gew.% enthalten. Die Emulgatoren können nichtionisch, anionisch, kationisch, amphoter oder zwitterionisch sein, wobei nichtionische Emulgatoren bevorzugt werden. Geeignet sind z.B.
- Ethoxylierungsprodukte von Fettalkoholen, Fettsäuren, Fettsäureglyceriden oder Alkylphenolen, insbesondere Anlagerungsprodukte von 2 bis 30 mol Ethylenoxid und/oder 1 bis 5 mol Propylenoxid an C8- bis C22-Fettalkohole, an C12- bis C22-Fettsäuren oder an Alkylphenole mit 8 bis 15 C-Atomen in der Alkylgruppe,
- C12- bis C22-Fettsäuremono- und -diester von Anlagerungsprodukten von 1 bis 30 mol Ethylenoxid an Glycerin,
- Anlagerungsprodukte von 5 bis 60 mol Ethylenoxid an Rizinusöl oder an gehärtetes (hydriertes) Rizinusöl.
- Mono-, Di- und/oder Triester der Phosphorsäure mit Anlagerungsprodukten von 2 bis 30 mol Ethylenoxid an C8- bis C22-Fettalkohole
- Fettsäurezuckerester, insbesondere Ester aus Saccharose und ein oder zwei C8- bis C22-Fettsäuren; z.B. Verbindungen mit den INCI-Bezeichnungen Sucrose Cocoate, Sucrose Dilaurate, Sucrose Distearate, Sucrose Laurate, Sucrose Myristate, Sucrose Oleate, Sucrose Palmitate, Sucrose Ricinoleate, Sucrose Stearate
- Ethoxylierte Sorbitanfettsäureester, insbesondere Ester aus Sorbitan und ein, zwei oder drei C8- bis C22-Fettsäuren und einem Ethoxylierungsgrad von 4 bis 20; z.B. Verbindungen mit den INCI-Bezeichnungen Polysorbatex, wobei x den Ethoxylierungsgrad angibt
- Polyglycerylfettsäureester, insbesondere aus ein, zwei oder mehreren C8- bis C22-Fettsäuren und Polyglycerin mit vorzugsweise 2 bis 20 Glyceryleinheiten.

Die Emulgatoren können bei Raumtemperatur flüssige oder feste Konsisitenz haben. In einer besonderen Ausführungsform haben die Emulgatoren wachsartige Konsistenz und einen Tropfpunkt oberhalb 25°C.

### Haarfestigende Polymere

In einer bevorzugten Ausführungsform enthält das erfindungsgemäße Haarwachsprodukt zusätzlich mindestens ein haarfestigendes Polymer. Die haarfestigenden Polymere sind vorzugsweise in einer Menge von 0 bis 15 Gew.%, 0,1 bis 10 Gew.% oder 0,5 bis 5 Gew.% enthalten.

Geeignete haarfestigende Polymere sind insbesondere die im International Cosmetic Ingredient Dictionary and Handbook, 9. Auflage, Seiten 2920-2921 unter der Funktion "Hair Fixatives" aufgeführten Polymere.

Das haarfestigende Polymer kann ausgewählt sein aus anionischen, kationischen, zwitterionischen, nichtionischen und amphoteren Polymeren. Es kann sich um natürliche oder um synthetische Polymere handeln. Unter synthetischen Polymeren werden solche Polymere verstanden, welche rein synthetischen, nicht natürlichen Ursprungs sind, insbesondere solche, die durch radikalische Polymerisation aus ethylenisch ungesättigten Monomeren oder durch Polykondensation herstellbar sind. Unter natürlichen Polymeren werden Polymere natürlichen Ursprungs verstanden, die auch nachträglich chemisch oder physikalisch modifiziert sein können. Bevorzugt sind insbesondere solche Polymere, die eine ausreichende Löslichkeit oder Dispergierbarkeit in dem Trägermedium, insbesondere in Wasser, Alkohol oder Wasser/ Alkohol-Gemischen besitzen, um in dem Trägermedium in gelöster oder homogen dispergierter Form vorzuliegen. Unter haarfestigenden Polymeren werden erfindungsgemäß solche Polymere verstanden, die bei Anwendung in 0,01 bis 5%iger wässriger, alkoholischer oder wässrig-alkoholischer Lösung oder Dispersion in der Lage sind, auf dem Haar einen Polymerfilm abzuscheiden und das Haar zu festigen.

Geeignete nichtionische Polymere sind Homo- oder Copolymere, die aus mindestens einem der folgenden Monomere aufgebaut sind: Vinyllactamen, insbesondere Vinylpyrrolidon und Vinylcaprolactam, Vinylestern wie z.B. Vinylacetat, Vinylalkoholen, Acrylamiden, Methacrylamiden, Alkylacrylamiden, Dialkylacrylamiden, Alkylmethacrylamiden, Dialkylmethacrylamiden, Dialkylaminoalkylmethacrylamiden, Dialkylaminoalkylacrylamiden, Alkylacrylaten, Alkylmethacrylaten, Propylenglykol oder Ethylenglykol, wobei die Alkylgruppen dieser Monomere vorzugsweise C1- bis C7-Alkylgruppen, besonders bevorzugt C1- bis C3-Alkylgruppen sind. Geeignet sind z.B. Homopolymere des Vinylcaprolactams, des Vinylpyrrolidons oder des N-Vinylformamids. Weitere geeignete synthetische filmbildende, nichtionische, haarfestigende Polymere sind z.B. Copolymerisate aus Vinylpyrrolidon und Vinylacetat, Terpolymere aus Vinylpyrrolidon, Vinylacetat und Vinylpropionat, Polyacrylamide; Polyvinylalkohole sowie Polyethylenglykol/Polypropylenglykol Copolymere. Besonders bevorzugt sind Polyvinylpyrrolidon, Polyvinylcaprolactam und deren Copolymere mit mindestens einem weiteren nichtionischen Monomer, insbesondere Polyvinylpyrrolidon/ Vinylacetat Copolymere.

Geeignete anionische haarfestigende Polymere sind z.B. synthetische Homo- oder Copolymere aus Säuregruppen enthaltenden Monomereinheiten, welche gegebenenfalls mit Comonomeren, die keine Säuregruppen enthalten, copolymerisiert sind. Die Säuregruppen sind vorzugsweise ausgewählt aus -COOH, -SO₃H, -OSO₃H, -OPO₂H und -OPO₃H₂, von denen die Carbonsäuregruppen bevorzugt sind. Die Säuregruppen können unneutralisiert, teilweise oder vollständig neutralisiert vorliegen. Sie liegen vorzugsweise zu 50 bis 100% in anionischer bzw. neutralisierter Form vor. Als Neutralisationsmittel können für kosmetische Zwecke geeignete organische oder anorganische Basen verwendet werden. Beispiele für Basen sind Aminoalkohole wie z.B. Aminomethylpropanol (AMP), Triethanolamin, Monoethanolamin oder Tetrahydroxypropylethylendiamin und Ammoniak, NaOH und andere. Geeignete Monomere sind ungesättigte, radikalisch polymerisierbare Verbindungen, welche mindestens eine Säuregruppe tragen, insbesondere Carboxyvinylmonomere. Geeignete Säuregruppen enthaltende Monomere sind z.B. Acrylsäure, Methacrylsäure, Crotonsäure, Maleinsäure bzw. Maleinsäureanhydrid oder deren Monoester.

Nicht mit Säuregruppen substituierte Comonomere sind z.B. Acrylamid, Methacrylamid, Alkyl- und Dialkylacrylamid, Alkyl- und Dialkylmethacrylamid, Alkylacrylat, Alkylmethacrylat, Vinylcaprolacton, Vinylpyrrolidon, Vinylester, Vinylalkohol, Propylenglykol oder Ethylenglykol, aminsubstituierte Vinylmonomere wie z.B. Dialkylaminoalkylacrylat, Dialkylaminoalkylmethacrylat, Monoalkylaminoalkylacrylat und Monoalkylaminoalkylmethacrylat, wobei die Alkylgruppen dieser Monomere vorzugsweise C1- bis C7-Alkylgruppen, besonders bevorzugt C1- bis C3-Alkylgruppen sind.

Geeignete anionische Polymere sind insbesondere Copolymere der Acrylsäure oder Methacrylsäure mit Monomeren ausgewählt aus Acrylsäure- oder Methacrylsäureestern, Acrylamiden, Methacrylamiden und Vinylpyrrolidon, Homopolymere der Crotonsäure sowie Copolymere der Crotonsäure mit Monomeren ausgewählt aus Vinylestern, Acrylsäure- oder Methacrylsäureestern, Acrylamiden und Methacrylamiden. Ein geeignetes natürliches Polymer ist beispielsweise Schellack. Bevorzugte anionische Polymere sind vernetzte oder unvernetzte Vinylacetat/Crotonsäure Copolymere. Ebenso bevorzugt sind partialveresterte Copolymere zwischen Vinylmethylether und Maleinsäureanhydrid. Weitere bevorzugte anionische Polymere sind z.B. Terpolymere aus Acrylsäure, Alkylacrylat und N-Alkylacrylamid, insbesondere Acrylsäure/ Ethylacrylat/N-t-Butylacrylamid Terpolymere oder Terpolymere aus Vinylacetat, Crotonat und Vinylalkanoat, insbesondere Vinylacetat/Crotonat/Vinylneodecanoat Copolymere; Copolymere aus Acryl- oder Methacrylsäure und Acryl- oder Methacrylsäurealkylestern, wobei die Alkylgruppen vorzugsweise 1 bis 7 C-Atome enthalten sowie Polystyrolsulfonate.

Geeignete haarfestigende amphotere oder zwitterionische Polymere sind Polymere, welche neben sauren oder anionischen Gruppen als weitere funktionelle Gruppen basische oder kationische Gruppen, insbesondere primäre, sekundäre, tertiäre oder quaternäre Amingruppen enthalten. Zwitterionische Polymer sind Polymere, welche aufgebaut sind aus mindestens einer Monomerart, welche sowohl quaternäre Amingruppen als auch Säuregruppen aufweist oder aus Polymeren, welche aufgebaut sind aus mindestens einer ersten Monomerart, welche quaternäre Amingruppen aufweist und mindestens einer zweiten Monomerart, welche Säuregruppen aufweist. Amphotere Polymere sind z.B. aufgebaut aus mindestens einer Monomerart, welche Säuregruppen aufweist und mindestens einer weiteren Monomerart, welche basische Amingruppen aufweist. Beispiele hierfür sind Copolymere gebildet aus Alkylacrylamid (insbesondere Octylacrylamid), Alkylaminoalkylmethacrylat (insbesondere t-Butylaminoethylmethacrylat) und zwei oder mehr Monomeren ausgewählt aus Acrylsäure, Methacrylsäure oder deren C1- bis C4-Alkylestern, wobei mindestens eines der Monomere eine Säuregruppe aufweist, wie sie z.B. unter dem Handelsnamen Amphomer^{®} oder Amphomer^{®} LV-71 erhältlich sind.

Weitere geeignete Polymere sind Copolymere von Acrylsäure, Methylacrylat und Methacrylamidopropyltrimethylammoniumchlorid (INCI-Bezeichnung: Polyquaternium-47), Copolymere aus Acrylamidopropyltrimethylammoniumchlorid und Acrylaten oder Copolymere aus Acrylamid, Acrylamidopropyltrimethylammoniumchlorid, 2-Amidopropylacrylamidsulfonat und Dimethylaminopropylamin (INCI-Bezeichnung: Polyquaternium-43). Geeignet sind auch Polymere mit Betaingruppen tragenden Monomeren wie z.B. Copolymere aus Methacryloylethylbetain und zwei oder mehr Monomeren von Acrylsäure oder deren einfachen Estern, bekannt unter der INCI-Bezeichnung Methacryloyl Ethyl Betaine/Acrylates Copolymer.

Geeignete kationische Polymere enthalten vorzugsweise quaternäre Amingruppen. Die kationischen Polymere können Homo- oder Copolymere sein, wobei die quaternären Stickstoffgruppen entweder in der Polymerkette oder vorzugsweise als Substituent an einem oder mehreren der Monomeren enthalten sind. Die Ammoniumgruppen enthaltenden Monomere können mit nicht kationischen Monomeren copolymerisiert sein. Geeignete kationische Monomere sind ungesättigte, radikalisch polymerisierbare Verbindungen, welche mindestens eine kationische Gruppe tragen, insbesondere ammoniumsubstituierte Vinylmonomere wie z.B. Trialkylmethacryloxyalkylammonium, Trialkylacryloxyalkylammonium, Dialkyldiallylammonium und quaternäre Vinylammoniummonomere mit cyclischen, kationische Stickstoffe enthaltenden Gruppen wie Pyridinium, Imidazolium oder quaternäre Pyrrolidone, z.B. Alkylvinylimidazolium, Alkylvinylpyridinium, oder Alkylvinylpyrrolidon Salze. Die Alkylgruppen dieser Monomere sind vorzugsweise niedere Alkylgruppen wie z.B. C1- bis C7-Alkylgruppen, besonders bevorzugt C1- bis C3-Alkylgruppen. Die Ammoniumgruppen enthaltenden Monomere können mit nicht kationischen Monomeren copolymerisiert sein. Geeignete Comonomere sind beispielsweise Acrylamid, Methacrylamid, Alkyl- und Dialkylacrylamid, Alkyl- und Dialkylmethacrylamid, Alkylacrylat, Alkylmethacrylat, Vinylcaprolacton, Vinylcaprolactam, Vinylpyrrolidon, Vinylester, z.B. Vinylacetat, Vinylalkohol, Propylenglykol oder Ethylenglykol, wobei die Alkylgruppen dieser Monomere vorzugsweise C1- bis C7- oder C1- bis C3-Alkylgruppen sind.

Kationische Polymere mit quaternären Amingruppen sind z.B. die im CTFA Cosmetic Ingredient Dictionary unter den Bezeichnungen Polyquaternium beschriebenen Polymere wie Methylvinylimidazoliumchlorid/Vinylpyrrolidon Copolymer (Polyquaternium-16) oder quaternisiertes Vinylpyrrolidon/Dimethylaminoethylmethacrylat Copolymer (Polyquaternium-11) sowie quaternäre Silikonpolymere bzw. -oligomere wie beispielsweise Silikonpolymere mit quaternären Endgruppen (Quaternium-80). Von den kationischen Polymeren ist z.B. Vinylpyrrolidon/Dimethylaminoethylmethacrylatmethosulfat Copolymer, das unter den Handelsbezeichnungen Gafquat^{®} 755 N und Gafquat^{®} 734 vertrieben wird, geeignet. Weitere kationische Polymere sind beispielsweise das unter dem Handelsnamen LUVIQUAT^{®} HM 550 vertriebene Copolymer aus Polyvinylpyrrolidon und Imidazoliminmethochlorid, das unter dem Handelsnamen Merquat^{®} Plus 3300 vertriebene Terpolymer aus Dimethyldiallylammoniumchlorid, Natriumacrylat und Acrylamid, das unter dem Handelsnamen Gaffix^{®} VC 713 vertriebene Terpolymer aus Vinylpyrrolidon, Dimethylaminoethylmethacrylat und Vinylcaprolactam und das unter dem Handelsnamen Gafquat^{®} HS 100 vertriebene Vinylpyrrolidon/Methacrylamidopropyltrimethylammoniumchlorid Copolymer.

Geeignete kationische Polymere, die von natürlichen Polymeren abgeleitet sind, sind kationische Derivate von Polysacchariden, beispielsweise kationische Derivate von Cellulose, Stärke oder Guar. Geeignet sind weiterhin Chitosan und Chitosanderivate. Kationische Polysaccharide haben die allgemeine Formel

G-O-B-N⁺R¹R²R³ X⁻

G ist ein Anhydroglucoserest, beispielsweise Stärke- oder Celluloseanhydroglucose;
B ist eine divalente Verbindungsgruppe, beispielsweise Alkylen, Oxyalkylen, Polyoxyalkylen oder Hydroxyalkylen;
R1, R2 und R3 sind unabhängig voneinander Alkyl, Aryl, Alkylaryl, Arylalkyl, Alkoxyalkyl oder Alkoxyaryl mit jeweils bis zu 18 C-Atomen, wobei die Gesamtzahl der C-Atome in R¹, R² und R³ vorzugsweise maximal 20 ist;
X ist ein übliches Gegenanion X⁻, beispielsweise ein Halogen, Acetat, Phosphat, Nitrat oder Alkylsulfat, vorzugsweise ein Chlorid. Eine kationische Cellulose wird unter der Bezeichnung Polymer JR von Amerchol vertrieben und hat die INCI-Bezeichnung Polyquaternium-10. Eine weitere kationische Cellulose trägt die INCI-Bezeichnung Polyquaternium-24 und wird unter dem Handelsnamen Polymer LM-200 von Amerchol vertrieben. Ein geeignetes kationisches Guarderivat wird unter der Handelsbezeichnung Jaguar^{®} R vertrieben und hat die INCI-Bezeichnung Guar Hydroxypropyltrimonium Chloride. Besonders bevorzugte kationaktive Stoffe sind Chitosan, Chitosansalze und Chitosan-Derivate. Chitosane sind vollständig oder partiell deacetylierte Chitine. Das Molekulargewicht des Chitosans kann über ein breites Spektrum verteilt sein, beispielsweise von 20.000 bis ca. 5 Millionen g/mol. Geeignet ist beispielsweise ein niedermolekulares Chitosan mit einem Molekulargewicht von 30.000 bis 70.000 g/mol. Vorzugsweise liegt das Molekulargewicht jedoch über 100.000 g/mol, besonders bevorzugt von 200.000 bis 700.000 g/mol. Der Deacetylierungsgrad beträgt vorzugsweise 10 bis 99%, besonders bevorzugt 60 bis 99%. Ein bevorzugtes Chitosansalz ist Chitosoniumpyrrolidoncarboxylat, welches beispielsweise unter der Bezeichnung Kytamer^{®} PC von der Firma Amerchol, USA, vertrieben wird. Das enthaltene Chitosan hat ein Molekulargewicht von ca. 200.000 bis 300.000 g/mol und ist zu 70 bis 85% entacetyliert. Als Chitosanderivate kommen quaternisierte, alkylierte oder hydroxyalkylierte Derivate, beispielsweise Hydroxyethyl-, Hydroxypropyl- oder Hydroxybutylchitosan in Betracht. Die Chitosane oder Chitosanderivate liegen vorzugsweise in neutralisierter oder partiell neutralisierter Form vor. Der Neutralisationsgrad für das Chitosan oder das Chitosanderivat liegt vorzugsweise bei mindestens 50%, besonders bevorzugt zwischen 70 und 100%, bezogen auf die Anzahl der freien Basengruppen. Als Neutralisationsmittel können prinzipiell alle kosmetisch verträglichen anorganischen oder organischen Säuren verwendet werden wie beispielsweise Ameisensäure, Äpfelsäure, Milchsäure, Pyrrolidoncarbonsäure, Salzsäure u.a., von denen die Pyrrolidoncarbonsäure und die Milchsäure besonders bevorzugt sind.

### Pigmente

In einer bevorzugten Ausführungsform enthält das erfindungsgemäße Haarwachsprodukt zusätzlich mindestens ein Pigment. Hierbei kann es sich um farbige Pigmente handeln, welche der Produktmasse oder dem Haar Farbeffekte verleihen oder es kann sich um Glanzeffektpigmente handeln, welche der Produktmasse oder dem Haar Glanzeffekte verleihen. Die Farb- oder Glanzeffekte am Haar sind vorzugsweise temporär, d.h. sie halten bis zur nächsten Haarwäsche und können durch Waschen der Haare mit üblichen Shampoos wieder entfernt werden. Die Pigmente liegen in der Produktmasse in ungelöster Form vor und können in einer Menge von 0,01 bis 25 Gew.%, besonders bevorzugt von 5 bis 15 Gew.% enthalten sein.

Bei den Pigmenten handelt es sich vorzugsweise nicht um Nano- sonder um Mikropigmente. Die bevorzugte Teilchengröße beträgt 1 bis 200 µm, insbesondere 3 bis 150 µm, besonders bevorzugt 10 bis 100 µm.

Die Pigmente sind im Anwendungsmedium praktisch unlösliche Farbmittel und können anorganisch oder organisch sein. Auch anorganisch-organische Mischpigmente sind möglich. Bevorzugt sind anorganische Pigmente. Der Vorteil der anorganischen Pigmente ist deren ausgezeichnete Licht-, Wetter- und Temperaturbeständigkeit. Die anorganischen Pigmente können natürlichen Ursprungs sein, beispielsweise hergestellt aus Kreide, Ocker, Umbra, Grünerde, gebranntem Terra di Siena oder Graphit. Bei den Pigmenten kann es sich um Weißpigmente wie z.B. Titandioxid oder Zinkoxid, um Schwarzpigmente wie z.B. Eisenoxidschwarz, Buntpigmente wie z.B. Ultramarin oder Eisenoxidrot, um Glanzpigmente, Metalleffekt-Pigmente, Perlglanzpigmente sowie um Fluoreszenz- oder Phosphoreszenzpigmente handeln, wobei vorzugsweise mindestens ein Pigment ein farbiges, nicht-weißes Pigment ist. Geeignet sind Metalloxide, -hydroxide und -oxidhydrate, Mischphasenpigmente, schwefelhaltige Silicate, Metallsulfide, komplexe Metallcyanide, Metallsulfate, -chromate und -molybdate sowie die Metalle selbst (Bronzepigmente). Geeignet sind insbesondere Titandioxid (CI 77891), schwarzes Eisenoxid (CI 77499), gelbes Eisenoxid (CI 77492), rotes und braunes Eisenoxid (CI 77491), Manganviolett (CI 77742), Ultramarine (Natrium-Aluminiumsulfosilikate, CI 77007, Pigment Blue 29), Chromoxidhydrat (CI77289), Eisenblau (Ferric Ferrocyanide, CI77510), Carmine (Cochineal).

Besonders bevorzugt sind Perlglanz- und Farbpigmente auf Mica- bzw. Glimmerbasis welche mit einem Metalloxid oder einem Metalloxychlorid wie Titandioxid oder Wismutoxychlorid sowie gegebenenfalls weiteren farbgebenden Stoffen wie Eisenoxiden, Eisenblau, Ultramarine, Carmine etc. beschichtet sind und wobei die Farbe durch Variation der Schichtdicke bestimmt sein kann. Derartige Pigmente werden beispielsweise unter den Handelsbezeichnungen Rona^{®} , Colorona^{®}, Dichrona^{®} und Timiron^{®} von der Firma Merck, Deutschland vertrieben.

Organische Pigmente sind beispielsweise die natürlichen Pigmente Sepia, Gummigutt, Knochenkohle, Kasseler Braun, Indigo, Chlorophyll und andere Pflanzenpigmente. Synthetische organische Pigmente sind beispielsweise Azo-Pigmente, Anthrachinoide, Indigoide, Dioxazin-, Chinacridon-, Phtalocyanin-, Isoindolinon-, Perylen- und Perinon-, Metallkomplex-, Alkaliblau- und Diketopyrrolopyrrol-Pigmente.

Das erfindungsgemäße Haarwachsprodukt kann auch anstelle von oder zusätzlich zu den Pigmenten gelöste Farbstoffe enthalten.

Das erfindungsgemäße Haarwachsprodukt ist vorzugsweise im wesentlichen wasserfrei, d.h. es enthält entweder kein oder nur geringe Mengen Wasser bis maximal 10 Gew.% oder weniger als 5 Gew.%.

Zusätzlich können auch geeignete hydrophobe, silikonfreie, weichwachsartige Stoffe in Mengen von z.B. 0,1 bis 10 Gew.% eingesetzt werden. Die Erstarrungspunkte dieser weichswachsartigen Stoffe liegen in der Regel im Bereich von ca. 25°C bis unter 40°C, z.B. halbfeste Paraffine, insbesondere Produkte mit der INCI-Bezeichnung Petrolatum, z.B. Vaseline. Hierbei handelt es sich um eine halbfeste Mischung von aus Petroleum gewonnenen Kohlenwasserstoffen.

### Zusatzstoffe

Zusätzlich zu den vorstehend genannten Inhaltsstoffen kann die Zusammensetzung weitere Zusatzstoffe enthalten:
- Lösungsmittel wie ein- oder mehrwertige C1- bis C4-Alkohole, insbesondere Ethanol, Isopropanol, Glycerine oder Glykole in einer Menge bis zu 15 Gew.%, vorzugsweise 0,1 bis 8 Gew.%
- Gelöste kosmetische Farbstoffe in einer Menge bis zu 6 Gew.%, vorzugsweise 0,1 bis 4 Gew.%
- Parfüm- und Duftstoffe in einer Menge von bis zu 2 Gew.%, vorzugsweise 0,01 bis 1 Gew.% oder 0,2 bis 0,5 Gew.%
- Konservierungsmittel in einer Menge von bis zu 1 Gew.%, vorzugsweise 0,01 bis 0,5 Gew.%, insbesondere Parahydroxybenzoesäureester, Benzoesäure, Salicylsäure, Sorbinsäure, Mandelsäure, Polyhexamethylen-biguanid-hydrochlorid oder Isothiazolinonderivate
- Haarpflegende Zusätze wie z.B. Betain, Panthenol, Menthol, Vitamine oder ähnliche Stoffe in einer Menge von bis zu 5 Gew.%, vorzugsweise 0,01 bis 4 Gew.% oder 0,1 bis 0,5 Gew.%;
- Lichtschutzstoffe, UV-Filter etc. in einer Menge von bis zu 5 Gew.%, vorzugsweise 0,01 bis 4 Gew.%.

### Bevorzugte Ausführungsform

Eine bevorzugte Ausführungsform ist ein Haarwachsprodukt mit einem Gehalt an
(A) 10 bis 40 Gew.% mindestens eines silikonfreien Wachses, ausgewählt aus tierischen Wachsen, pflanzlichen Wachsen, mineralischen Wachsen, synthetischen Wachsen, mikrokristallinen Wachsen, makrokristallinen Wachsen, Paraffinwachsen, Ozokerit, Montanwachsen, Fischer-Tropsch-Wachsen, Polyolefinwachsen, Bienenwachs, Wollwachs, Wollwachsalkoholen, Candelillawachs, Carnaubawachs, Japanwachs, gehärteten Fetten, Fettsäureestern und Fettsäureglyceriden;
(B) 20 bis 80 Gew.% mindestens einer, bei 25°C flüssigen Silikonverbindung, ausgewählt aus linearen Polydimethylsiloxanen, cyclischen Polydimethylsiloxanen, hydroxysubstituierten Polydimethylsiloxanen, aminosubstituierten Silikonen und mit aromatischen Gruppen substituierten Siloxanen;
(C) 5 bis 40 Gew.% mindestens eines Silikonwachses mit einem Schmelzpunkt im Bereich von 20 bis 45 °C, ausgewählt aus Alkylmethyl-dimethylsiloxanen, wobei die Alkylgruppe mindestens 8 C-Atome aufweist;
(D) 0,1 bis 30 Gew.% mindestens eines Emulgators;
(E) 0 bis 15 Gew.% mindestens eines haarfestigenden Polymers;
(F) 0 bis 10 Gew.% Wasser.

### Herstellverfahren

Erfindungsgemäße Haarwachsprodukte können hergestellt werden, indem die festen Wachskomponenten aufgeschmolzen und mit den übrigen, nicht-flüchtigen Inhaltsstoffen vermischt werden. Anschließend wird abgekühlt und kurz vor dem Erstarren werden gegebenenfalls leichtflüchtige Zusatzstoffe zugegeben und vermischt. Die noch fließfähige Masse wird vor dem Erstarren in die gewünschte Verpackung, z.B. Kunststofftiegel abgefüllt.

### Anwendungsverfahren

Gegenstand der Erfindung ist die Verwendung einer erfindungsgemäßen, festen Zusammensetzung zur Haarbebehandlung.

Das Produkt wird bevorzugt auf trockenem Haar angewendet. Eine Menge, die in Abhängigkeit der Haarlänge varriieren kann, z.B. von ungefähr Erbsengröße bis ungefähr Haselnussgröße wird mit den Fingern entnommen. Das Wachs wird in den Handflächen verrieben und durch die Handwärme und durch die Verreibung erfolgende Scherung aufgeschmolzen oder zumindest stark erweicht. Im erweichten oder mehr oder weniger flüssigen Zustand wird das Wachs in das Haar eingearbeitet und die gewünschte Frisur erstellt. Es können auch separate Haarsträhnen oder Locken einzeln behandelt werden, um diese zu akzentuieren. Im Haar erhärtet das Wachs und die gestaltete Frisur erhält Stabilität, Glanz, Texture und Halt.

Das erfindungsgemäße Haarwachsprodukt ermöglicht aufgrund der wachsartigen Konsistenz und seiner kohäsiven Eigenschaften eine individuelle Frisurengestaltung sowie die gezielte Behandlung einzelner Haarsträhnen. Die Produktmasse ist leicht auf dem Haar verteilbar. Das behandelte Haar zeichnet sich durch einen ausgeprägten Glanz sowie durch eine hohe Formstabilität der erstellten Frisur aus. Das Haar wird dabei nicht übermäßig belastet und die Produktmasse ist leicht auswaschbar. Das Produkt zeichnet sich im Vergleich zu herkömmlichen Produkten durch eine reduzierte Klebrigkeit der behandelten Haare aus.

Die nachfolgenden Beispiele sollen den Gegenstand der Erfindung näher erläutern.

### Beispiele

### Beispiel 1

| | |
|---|---|
| 20 g | Kohlenwasserstoffwachs (Ozokerit) |
| 48 g | Silikonöle (Dimethylpolysiloxan, Phenyltrimethicone, Dimethiconol) |
| 15 g | alkylmodifiziertes Silikonwachs mit Schmelzpunkt ca. 30°C (Stearyl Dimethicone) |
| q.s. | Parfüm |

### Beispiel 2

| | |
|---|---|
| 20 g | Kohlenwasserstoffwachs (Ceresin) |
| 48 g | Silikonöle (Dimethylpolysiloxan, Phenyltrimethicone, Dimethiconol) |
| 15 g | alkylmodifiziertes Silikonwachs mit Schmelzpunkt ca. 30°C (Stearyl Dimethicone) |
| 16 g | Emulgator |
| q.s. | Parfüm |

### Beispiel 3

| | A | B |
|---|---|---|
| Polydimethylsiloxan | 44 g | 44 g |
| Ceresin, Tropfpunkt 76-84°C | 20 g | 35 g |
| Stearyl Dimethicone | 15 g | - |
| Ceteareth-25 | 8 g | 8 g |
| Oleth-3 | 5 g | 5 g |
| Phenyltrimethicone | 3 g | 3 g |
| PPG-1-PEG-9 Lauryl Glycol Ether | 3 g | 3 g |
| Dimethiconol | 1,8 g | 1,8 g |

Die Inhaltstoffe der Zusammensetzungen werden bei erhöhter Temperatur aufgeschmolzen, miteinander vermischt, in einen Kunststofftiegel abgefüllt und abkühlen gelassen.

Die erfindungsgemäße Zusammenstzung A wird hinsichtlich ihrer Anwendungseingeschaften mit der nicht erfindungsgemäßen Vergleichszusammensetzung B durch eine sensorische Überprüfung durch Anfühlen verglichen. Haare, die mit Zusammensetzung A behandelt sind, weisen eine deutlich geringere Klebrigkeit auf als Haare, die mit Zusammensetzung B behandelt sind.

Die erfindungsgemäßen Zusammensetzungen zeichnen sich aus durch eine gute Verteilbarkeit, eine gute Einarbeitbarkeit in das Haar, eine gute Auswaschbarkeit, gute Struktur und Glanz der Haare, einen anti-Frizz Effekt, einen natürlichen Halt der Haare, einen glatten Griff der Haare, eine gute Modellierbarkeit von Locken und eine gute Akzentuierbarkeit einzelner Haarsträhnen.

## Patentansprüche

1. Verwendung einer Zusammensetzung mit einem Gehalt an
(A) mindestens einem silikonfreien Wachs.
(B) mindestens einem, bei 25°C flüssigen hydrophoben Öl
(C) mindestens einem Silikonwachs mit einem Schmelzpunkt im Bereich von 20 bis 45 °C als Festes Haarwachsprodukt zur Behandlung oder Erstellung der menschlichen Frisur.

2. Verwendung nach Anspruch 1, **dadurch gekennzeichnet, dass** das silikonfreie Wachs einen Erstarrungspunkt oberhalb 40°C aufweist und ausgewählt ist aus tierischen Wachsen, pflanzlichen Wachsen, mineralischen Wachsen, synthetischen Wachsen, mikrokristallinen Wachsen, makrokristallinen Wachsen, Paraffinwachsen, Ozokerit, Montanwachsen, Fischer-Tropsch-Wachsen, Polyolefinwachsen, Bienenwachs, Wollwachs, Wollwachsalkoholen, Candelillawachs, Carnaubawachs, Japanwachs, gehärteten Fetten, Fettsäureestern und Fettsäureglyceriden.

3. Verwendung nach Anspruch 2, **dadurch gekennzeichnet, dass** das silikonfreie Wachs ein Kohlenwasserstoffwachs ist.

4. Verwendung nach einem der vorhergehenden Ansprüche, **dadurch gekennzeichnet, dass** das hydrophobe Öl eine flüssige Silikonverbindung ist, ausgewählt aus linearen Polydimethylsiloxanen, cyclischen Polydimethylsiloxanen, hydroxysubstituierten Polydimethylsiloxanen, aminosubstituierten Silikonen und mit aromatischen Gruppen substituierte Siloxane.

5. Verwendung nach einem der vorhergehenden Ansprüche, **dadurch gekennzeichnet, dass** das Silikonwachs einen Schmelzpunkt im Bereich von 20 bis 40 °C aufweist und ausgewählt ist aus Alkylmethyl-dimethylsiloxanen, wobei die Alkylgruppe mindestens 8 C-Atome aufweist.

6. Verwendung nach Anspruch 6, **dadurch gekennzeichnet, dass** das Silikonwachs ein Stearylmethyl-dimethylsioxan ist.

7. Verwendung nach einem der vorhergehenden Ansprüche, **dadurch gekennzeichnet, dass** das Haarwachsprodukt zusätzlich mindestens einen Emulgator (D) enthält.

8. Verwendung nach Anspruch 7, **dadurch gekennzeichnet, dass** der Emulgator (D) ausgewählt ist aus
a) ethoxylierten Fettalkoholen, ethoxylierten Fettsäuren, ethoxylierten Fettsäureglyceriden oder ethoxylierten Alkylphenolen,
b) C12- bis C22-Fettsäuremono- und -diestern von Anlagerungsprodukten von 1 bis 30 mol Ethylenoxid an Glycerin,
c) Anlagerungsprodukten von 5 bis 60 mol Ethylenoxid an Rizinusöl oder an gehärtetes (hydriertes) Rizinusöl,
d) Mono-, Di- und Triestern der Phosphorsäure mit Anlagerungsprodukten von 2 bis 30 mol Ethylenoxid an C8- bis C22-Fettalkohole,
e) Fettsäurezuckerestern,
f) ethoxylierten Sorbitanfettsäureestern,
g) Polyglycerylfettsäureestem
oder aus Gemischen dieser Emulgatoren.

9. Verwendung nach einem der vorhergehenden Anspruche, **dadurch gekennzeichnet, dass** das Haarwachsprodukt wasserfrei ist oder maximal 10 Gew.% Wasser enthält.

10. Verwendung nach einem der vorhergehenden Ansprüche, **dadurch gekennzeichnet, dass** das Haarwachsprodukt zusätzlich mindestens ein haarfestigendes Polymer (E) enthält.

11. Verwendung nach Anspruch 10, **dadurch gekennzeichnet, dass** das haarfestigende Polymer (E) ausgewählt ist
- aus Polymeren, welche aufgebaut sind aus mindestens einem Monomer, ausgewählt aus Vinyllactamen, Vinylestern, Vinylalkoholen, Acrylamiden, Methacrylamiden, Alkylacrylamiden, Dialkylacrylamiden, Alkylmethacrylamiden, Dialkylmethacrylamiden, Dialkylaminoalkylmethacrylamiden, Dialkylaminoalkylacrylamiden, Alkylacrylaten, Alkylmethacrylaten, Propylenglykol oder Ethylenglykol;
- aus Polymeren, welche aufgebaut sind aus mindestens einem Monomer, ausgewählt aus Acrylsäure, Methacrylsäure, Crotonsäure, Maleinsäure, Maleinsäureanhydrid, Monoestern der Maleinsäure;
- aus Polymeren, welche aufgebaut sind aus mindestens einem Monomer, ausgewählt aus Trialkylmethacryloxyalkylammonium, Trialkylacryloxyalkylammonium, Dialkyldiallylammonium, Alkylvinylimidazolium, Alkylvinylpyridinium oder Alyklvinylpyrrolidon;
- aus Polymeren, welche aufgebaut sind aus mindestens einer Monomerart, welche Säuregruppen aufweist und mindestens einer weiteren Monomerart, welche basische Amingruppen aufweist;
- aus Polymeren, welche aufgebaut sind aus mindestens einer Monomerart, welche sowohl quaternäre Amingruppen als auch Säuregruppen aufweist oder aus Polymeren, welche aufgebaut sind aus mindestens einer ersten Monomerart, welche quaternäre Amingruppen aufweist und mindestens einer zweiten Monomerart, welche Säuregruppen aufweist.

12. Verwendung nach einem der vorhergehenden Ansprüche mit einem Gehalt des Haarwachsproduktes an
(A) 10 bis 40 Gew.% mindestens eines silikonfreien Wachses, ausgewählt aus tierischen Wachsen, pflanzlichen Wachsen, mineralischen Wachsen, synthetischen Wachsen, mikrokristallinen Wachsen, makrokristallinen Wachsen, Paraffinwachsen, Ozokerit, Montanwachsen, Fischer-Tropsch-Wachsen, Polyolefinwachsen, Bienenwachs, Wollwachs, Wollwachsalkoholen, Candelillawachs, Carnaubawachs, Japanwachs, gehärteten Fetten, Fettsäureestern und Fettsäureglyceriden;
(B) 20 bis 80 Gew.% mindestens einer, bei 25°C flüssigen Silikonverbindung, ausgewählt aus linearen Polydimethylsiloxanen, cyclischen Polydimethylsiloxanen, hydroxysubstituierten Polydimethylsiloxanen, aminosubstituierten Silikonen und mit aromatischen Gruppen substituierten Siloxanen;
(C) 5 bis 40 Gew.% mindestens eines Silikonwachses mit einem Schmelzpunkt im Bereich von 20 bis 45 °C, ausgewählt aus Alkylmethyl-dimethylsiloxanen, wobei die Alkylgruppe mindestens 8 C-Atome aufweist;
(D) 0,1 bis 30 Gew.% mindestens eines Emulgators;
(E) 0 bis 15 Gew.% mindestens eines haarfestigenden Polymers;
(F) 0 bis 10 Gew.% Wasser.

13. Verwendung nach einem der vorhergehenden Ansprüche, **dadurch gekennzeichnet, dass** der Erstarrungspunkt des Haarwachsprodukts größer oder gleich 30°C ist und die Nadelpenetrationszahl bei 20°C im Bereich von 10 bis 60 liegt.

14. Verwendung nach einem der vorhergehenden Ansprüche, **dadurch gekennzeichnet, dass** das Haarwachsproduktes es zusätzlich mindestens ein Pigment enthält.

## Claims

1. Use of a composition with a content of
(A) at least one silicone-free wax,
(B) at least one hydrophobic oil liquid at 25°C,
(C) at least one silicone wax with a melting point in the range from 20 to 45°C
as solid hair wax product for the treatment or creation of a person's hairstyle.

2. Use according to Claim 1, **characterized in that** the silicone-free wax has a solidification point above 40°C and is selected from animal waxes, vegetable waxes, mineral waxes, synthetic waxes, microcrystalline waxes, macrocrystalline waxes, paraffin waxes, ozokerite, montan waxes, Fischer-Tropsch waxes, polyolefin waxes, beeswax, wool wax, wool wax alcohols, candelilla wax, carnauba wax, Japan wax, hardened fats, fatty acid esters and fatty acid glycerides.

3. Use according to Claim 2, **characterized in that** the silicone-free wax is a hydrocarbon wax.

4. Use according to one of the preceding claims, **characterized in that** the hydrophobic oil is a liquid silicone compound selected from linear polydimethylsiloxanes, cyclic polydimethylsiloxanes, hydroxy-substituted polydimethylsiloxanes, amino-substituted silicones and siloxanes substituted by aromatic groups.

5. Use according to one of the preceding claims, **characterized in that** the silicone wax has a melting point in the range from 20 to 40°C and is selected from alkylmethyl-dimethylsiloxanes, where the alkyl group has at least 8 carbon atoms.

6. Use according to Claim 6, **characterized in that** the silicone wax is a stearylmethyl-dimethylsiloxane.

7. Use according to one of the preceding claims, **characterized in that** the hair wax product additionally comprises at least one emulsifier (D).

8. Use according to Claim 7, **characterized in that** the emulsifier (D) is selected from
a) ethoxylated fatty alcohols, ethoxylated fatty acids, ethoxylated fatty acid glycerides or ethoxylated alkylphenols,
b) C12- to C22-fatty acid mono- and diesters of addition products of from 1 to 30 mol of ethylene oxide onto glycerol,
c) addition products of from 5 to 60 mol of ethylene oxide onto castor oil or onto hardened (hydrogenated) castor oil,
d) mono-, di- and triesters of phosphoric acid with addition products of from 2 to 30 mol of ethylene oxide onto C8- to C22-fatty alcohols,
e) fatty acid sugar esters,
f) ethoxylated sorbitan fatty acid esters,
g) polyglyceryl fatty acid esters
or from mixtures of these emulsifiers.

9. Use according to one of the preceding claims, **characterized in that** the hair wax product is anhydrous or comprises at most 10% by weight of water.

10. Use according to one of the preceding claims, **characterized in that** the hair wax product additionally comprises at least one hair-setting polymer (E).

11. Use according to Claim 10, **characterized in that** the hair-setting polymer (E) is selected
- from polymers which are constructed from at least one monomer selected from vinyllactams, vinyl esters, vinyl alcohols, acrylamides, methacrylamides, alkylacrylamides, dialkylacrylamides, alkylmethacrylamides, dialkylmethacrylamides, dialkylaminoalkylmethacrylamides, dialkylaminoalkylacrylamides, alkyl acrylates, alkyl methacrylates, propylene glycol or ethylene glycol;
- from polymers which are constructed from at least one monomer selected from acrylic acid, methacrylic acid, crotonic acid, maleic acid, maleic anhydride, monoesters of maleic acid;
- from polymers which are constructed from at least one monomer selected from trialkylmethacryloxyalkylammonium, trialkylacryloxyalkylammonium, dialkyldiallylammonium, alkylvinylimidazolium, alkylvinylpyridinium or alkylvinylpyrrolidone;
- from polymers which are constructed from at least one type of monomer which has acid groups and at least one further type of monomer which has basic amine groups;
- from polymers which are constructed from at least one type of monomer which has both quaternary amine groups and acid groups, or from polymers which are constructed from at least one first type of monomer which has quaternary amine groups and at least one second type of monomer which has acid groups.

12. Use according to one of the preceding claims with a content in the hair wax product of
(A) 10 to 40% by weight of at least one silicone-free wax selected from animal waxes, vegetable waxes, mineral waxes, synthetic waxes, microcrystalline waxes, macrocrystalline waxes, paraffin waxes, ozokerite, montan waxes, Fischer-Tropsch waxes, polyolefin waxes, beeswax, wool wax, wool wax alcohols, candelilla wax, carnauba wax, Japan wax, hardened fats, fatty acid esters and fatty acid triglycerides;
(B) 20 to 80% by weight of at least one silicone compound liquid at 25°C selected from linear polydimethylsiloxanes, cyclic polydimethylsiloxanes, hydroxy-substituted polydimethylsiloxanes, amino-substituted silicones and siloxanes substituted with aromatic groups;
(C) 5 to 40% by weight of at least one silicone wax with a melting point in the range from 20 to 45°C, selected from alkylmethyl-dimethylsiloxanes, where the alkyl group has at least 8 carbon atoms;
(D) 0.1 to 30% by weight of at least one emulsifier;
(E) 0 to 15% by weight of at least one hair-setting polymer;
(F) 0 to 10% by weight of water.

13. Use according to one of the preceding claims, **characterized in that** the solidification point of the hair wax product is greater than or equal to 30°C and the needle penetration value at 20°C is in the range from 10 to 60.

14. Use according to one of the preceding claims, **characterized in that** the hair wax product additionally comprises at least one pigment.

## Revendications

1. Utilisation d'une composition ayant une teneur en
(A) au moins une cire exempte de silicone,
(B) au moins une huile hydrophobe liquide à 25 °C,
(C) au moins une cire de silicone ayant un point de fusion dans la plage de 20 à 45 °C,
en tant que produit capillaire solide à base de cire destiné au traitement ou à l'élaboration de la coiffure humaine.

2. Utilisation selon la revendication 1, **caractérisée en ce que** la cire exempte de silicone a un point de solidification supérieur à 40 °C et est choisie parmi des cires animales, des cires végétales, des cires minérales, des cires synthétiques, des cires microcristallines, des cires macrocristallines, des cires paraffiniques, l'ozokérite, les cires de lignite, les cires de Fischer-Tropsch, les cires polyoléfiniques, la cire d'abeille, la lanoline, les alcolanums, la cire de candelilla, la cire de carnauba, la cire du Japon, des graisses durcies, des esters d'acides gras et des glycérides d'acides gras.

3. Utilisation selon la revendication 2, **caractérisée en ce que** la cire exempte de silicone est une cire d'hydrocarbure.

4. Utilisation selon l'une quelconque des revendications précédentes, **caractérisée en ce que** l'huile hydrophobe est un composé silicone liquide choisi parmi des polydiméthylsiloxanes linéaires, des polydiméthylsiloxanes cycliques, des polydiméthylsiloxanes substitués par hydroxy, des silicones substitués par amino, et des siloxanes substitués par des groupes aromatiques.

5. Utilisation selon l'une quelconque des revendications précédentes, **caractérisée en ce que** la cire de silicone a un point de fusion dans la plage de 20 à 40 °C et est choisie parmi des alkylméthyl-diméthylsiloxanes dans lesquels le groupe alkyle a au moins 8 atomes de carbone.

6. Utilisation selon la revendication 6, **caractérisée en ce que** la cire de silicone est un stéarylméthyl-diméthylsiloxane.

7. Utilisation selon l'une quelconque des revendications précédentes, **caractérisée en ce que** le produit capillaire à base de cire en outre contient au moins un émulsifiant (D).

8. Utilisation selon la revendication 7, **caractérisée en ce que** l'émulsifiant (D) est chois parmi
a) des alcools gras éthoxylés, des acides gras éthoxylés, des glycérides d'acides gras éthoxylés ou des alkylphénols éthoxylés,
b) des mono- et diesters d'acides gras en C₁₂-C₂₂ de produits de fixation par addition de 1 à 30 moles d'oxyde d'éthylène sur le glycérol,
c) des produits de fixation par addition de 5 à 60 moles d'oxyde d'éthylène sur l'huile de ricin ou sur l'huile de ricin durcie (hydrogénée),
d) des mono-, di- et/ou triesters de l'acide phosphorique avec des produits de fixation par addition de 2 à 30 moles d'oxyde d'éthylène sur des alcools gras en C₈-C₂₂,
e) des esters glycidiques d'acides gras,
f) des esters éthoxylés d'acides gras et de sorbitanne
g) des polyglycérylesters d'acides gras
ou des mélanges de ces émulsifiants.

9. Utilisation selon l'une quelconque des revendications précédentes, **caractérisée en ce que** le produit capillaire à base de cire est anhydre ou contient au maximum 10% en poids d'eau.

10. Utilisation selon l'une quelconque des revendications précédentes, **caractérisée en ce que** le produit capillaire à base de cire en outre contient au moins un polymère (E) fixant les cheveux.

11. Utilisation selon la revendication 10, **caractérisée en ce que** le polymère (E) fixant les cheveux est choisi
- parmi des polymères qui sont formés à partir d'au moins un monomère choisi parmi des vinyllactames, des esters vinyliques, des alcools vinyliques, des acrylamides, des méthacrylamides, des alkylacrylamides, des dialkylacrylamides, des alkylméthacrylamides, des dialkylméthacrylamides des dialkylaminoalkylméthacrylamides, des dialkylaminoalkylacrylamides, des acrylates d'alkyle, des méthacrylates d'alkyle, le propylèneglycol ou l'éthylèneglycol;
- parmi des polymères qui sont formés à partir d'au moins un monomère choisi parmi l'acide acrylique, l'acide méthacrylique, l'acide crotonique, l'acide maléique, l'anhydride maléique, des monoesters de l'acide maléique ;
- parmi des polymères qui sont formés à partir d'au moins un monomère choisi parmi un trialkylméthacryloxyalkylammonium, un trialkylacryloxyalkylammonium, un dialkyldiallylammonium, un alkylvinylimidazolium, un alkylvinylpyridinium ou une alkylvinylpyrrolidone;
- parmi des polymères qui sont formés à partir d'au moins un type de monomère qui comporte des groupes acides et d'au moins un autre type de monomère qui comporte des groupes amino basiques;
- parmi des polymères qui sont formés à partir d'au moins un type de monomère qui comporte aussi bien des groupes amino quaternaires que des groupes acides ou parmi des polymères qui sont formés à partir d'au moins un premier type de monomère qui comporte des groupes amino quaternaires et d'au moins un deuxième type de monomère qui comporte des groupes acides.

12. Utilisation selon l'une quelconque des revendications précédentes du produit capillaire solide à base de cire ayant une teneur
(A) de 10 à 40 % en poids en au moins une cire exempte de silicone, choisie parmi des cires animales, des cires végétales, des cires minérales, des cires synthétiques, des cires microcristallines, des cires macrocristallines, des cires paraffiniques, l'ozokérite, les cires de lignite, les cires de Fischer-Tropsch, les cires polyoléfiniques, la cire d'abeille, la lanoline, les alcolanums, la cire de candelilla, la cire de carnauba, la cire du Japon, des graisses durcies, des esters d'acides gras et des glycérides d'acides gras ;
(B) de 20 à 80 % en poids en au moins un composé silicone liquide à 25 °C, choisi parmi des polydiméthylsiloxanes linéaires, des polydiméthylsiloxanes cycliques, des polydiméthylsiloxanes substitués par hydroxy, des silicones substitués par amino, et des siloxanes substitués par des groupes aromatiques ;
(C) de 5 à 40 % en poids en au moins une cire de silicone ayant un point de fusion dans la plage de 20 à 45 °C, choisie parmi des alkylméthyl-diméthylsiloxanes dans lesquels le groupe alkyle a au moins 8 atomes de carbone ;
(D) de 0,1 à 30 % en poids en au moins un émulsifiant ;
(E) de 0 à 15 % en poids en au moins un polymère fixant les cheveux ;
(F) de 0 à 10 % en poids en eau.

13. Utilisation selon l'une quelconque des revendications précédentes, **caractérisée en ce que** le point de solidification du produit capillaire à base de cire est supérieur ou égal à 30 °C et l'indice de pénétration de l'aiguille à 20 °C se situe dans la plage allant de 10 à 60.

14. Utilisation selon l'une quelconque des revendications précédentes, **caractérisée en ce que** le produit capillaire à base de cire en outre contient au moins un pigment.
